# EUROPEAN PATENT APPLICATION

(11) **EP 4 741 378 A1**
(43) Date of publication of application: **13.05.2026**
(21) Application number: 25202748.7
(22) Date of filing: 17.09.2025
(51) Int. Cl.: C07D 233/58, C07B 61/00

(54) **PREPARATION METHOD OF IONIC LIQUID**

(30) Priority: 08.11.2024 TW 113143005
(71) Applicant: Hon Hai Precision Industry Co., Ltd., 236038 New Taipei City (TW); SolidEdge Solution Inc., 300094 Hsinchu City (TW)
(72) Inventor: CHEN, Wei-Chao, 300094 Hsinchu City (TW); CHEN, Hsuan-Yu, 300094 Hsinchu City (TW); WANG, Pin-Han, 300094 Hsinchu City (TW); CHANG, Tseng-Lung, 300094 Hsinchu City (TW)
(74) Representative: Winter, Brandl - Partnerschaft mbB

(57) **Abstract**

A method of preparing an ionic liquid includes the following steps. A halogen-containing compound is reacted with a first compound to form a second compound. The halogen-containing compound includes a halohydrocarbon, a sulfonyl halide, or a combination thereof. The first compound includes an amine compound having a tertiary amine group, a phosphine compound, or a combination thereof. The second compound includes a first quaternary ammonium salt, a first quaternary phosphonium salt, or a combination thereof. The second compound and a lithium salt are reacted in a microwave device to form a third compound. The third compound includes a second quaternary ammonium salt, a second quaternary phosphonium salt, or a combination thereof. Anions of the second compound and the third compound are different. A microwave power of the microwave device is 700 watts to 1400 watts.

## Description

### BACKGROUND

### Field of Disclosure

The present disclosure relates to a method of preparing an ionic compound.

### Description of Related Art

An electrolyte is indispensable in batteries. Traditionally, liquid electrolytes are often used as electrolytes in the batteries, in which the liquid electrolytes such as ethylene carbonate, diethyl carbonate, methyl ethyl carbonate, and so on have low ignition point and volatility point. Therefore, when the battery thermally runs out of control, it can easily trigger safety concerns.

Since the liquid electrolytes have the above problems, other types of electrolytes or electrolyte auxiliary agents are gradually developed, such as solid electrolytes, special additives, gelled electrolytes, or flame retardants. The solid electrolytes have complex interface and ionic conductivity issues, so they are not widely used. Although the special additives such as fluoroethylene carbonate, difluoroethylene carbonate, and 2,2,2-trifluoroethylmethyl carbonate have high ignition and boiling points, the special additives are expensive and difficult to be produced. Therefore, the special additives are not widely used. Due to the introduction of gelled substances, the gelled electrolytes are less likely to produce a large number of reaction interfaces, and boiling issue still exists. The flame retardants have problems such as increased battery internal resistance and poor reaction uniformity after the flame retardants introducing into the battery, so they cannot be widely used. Therefore, there is an urgent need to develop other electrolytes that can improve flame resistance, battery stability, and ionic conductivity.

### SUMMARY

The present disclosure provides a method of preparing an ionic liquid, and the method includes the following steps. A halogen-containing compound is reacted with a first compound to form a second compound, in which the halogen-containing compound includes a halohydrocarbon, a sulfonyl halide, or a combination thereof, the first compound includes an amine compound having a tertiary amine group, a phosphine compound, or a combination thereof, and the second compound includes a first quaternary ammonium salt, a first quaternary phosphonium salt, or a combination thereof. The second compound is reacted with a lithium salt in a microwave device to form a third compound, in which the third compound includes a second quaternary ammonium salt, a second quaternary phosphonium salt, or a combination thereof, anions of the second compound and the third compound are different, and a microwave power of the microwave device is 700 watts to 1400 watts.

In some embodiments, the amine compound having the tertiary amine group includes a structure as shown in formula (1-1), an imidazole compound, a pyrrolidine compound, a pyridine compound, a pyrrole compound, a pyrazole compound, a structure as shown in formula (1-2), an imidazoline compound, a pyrazoline compound, or combinations thereof, 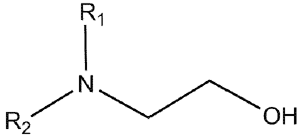 formula (1-1), 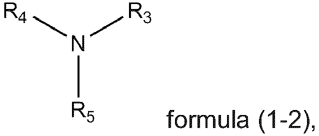 **in which** R₁ to R₅ are independently a C1 to C22 linear alkyl group, a C1 to C22 branched alkyl group, a C4 to C6 cycloalkyl group, or a C4 to C6 heterocycloalkyl group.

In some embodiments, the imidazole compound has a structure as shown in formula (1-3), 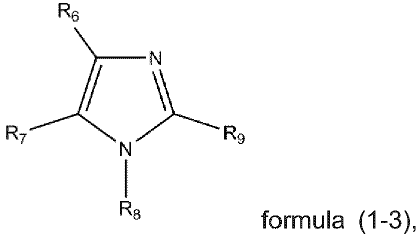 in which R₆ to R₉ are independently hydrogen, a C1 to C22 linear alkyl group, a C1 to C22 branched alkyl group, a C4 to C6 cycloalkyl group, or a C4 to C6 heterocycloalkyl group.

In some embodiments, the pyrrolidine compound has a structure as shown in formula (1-4), 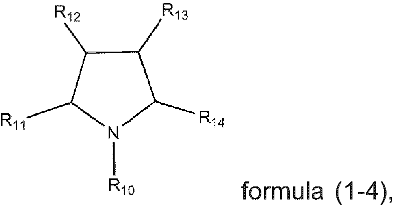 **in which** R₁₀ is a C1 to C22 linear alkyl group, a C1 to C22 branched alkyl group, a C4 to C6 cycloalkyl group, or a C4 to C6 heterocycloalkyl group, and R₁₁ to R₁₄ are independently hydrogen, a C1 to C22 linear alkyl group, a C1 to C22 branched alkyl group, a C4 to C6 cycloalkyl group, or a C4 to C6 heterocycloalkyl group.

In some embodiments, the halohydrocarbon has a structure as shown in formula (2-1), and the sulfonyl halide has a structure as shown in formula (2-2),

R₁'-X₁ formula (2-1),

R₂'-SO₂X₂ formula (2-2),

in which R₁' and R₂' are independently a C1 to C22 linear alkyl group, a C1 to C22 branched alkyl group, a C4 to C6 cycloalkyl group, a C4 to C6 heterocycloalkyl group, or a C6 to C10 aryl group, and X₁ and X₂ are independently fluorine, chlorine, bromine, or iodine.

In some embodiments, a reaction pressure of reacting the second compound with the lithium salt in the microwave device is greater than 1 atm and less than or equal to 2 atm.

In some embodiments, a reaction temperature of reacting the second compound with the lithium salt in the microwave device is 60 °C to 250 °C.

In some embodiments, the lithium salt includes lithium bis(trifluoromethanesulfonyl)imide, lithium bis(fluorosulfonyl)imide, lithium tetrafluoroborate, lithium hexafluorophosphate, lithium trifluoromethanesulfonate, lithium acetate, a structure as shown in formula (3-1), a structure as shown in formula (3-2), a structure as shown in formula (3-3), or combinations thereof, 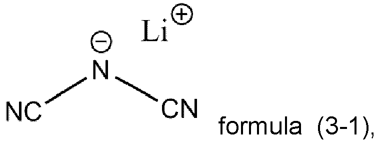 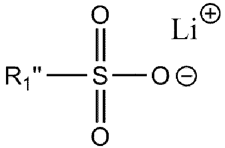 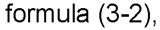 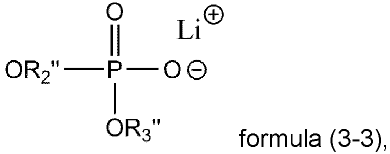 in which R₁", R₂", and R₃" are independently a C1 to C22 linear alkyl group, a C1 to C22 branched alkyl group, a C4 to C6 cycloalkyl group, or a C4 to C6 heterocycloalkyl group.

In some embodiments, a molar ratio of the halogen-containing compound to the first compound is 1:1 to 2:1, and a molar ratio of the second compound to the lithium salt is 1:1 to 1:2.

In some embodiments, the method of preparing the ionic liquid further includes after reacting the second compound with the lithium salt in the microwave device, performing a heating operation, in which a heating temperature of the heating operation is 60 °C to 180 °C.

In some embodiments, a heating time of the heating operation is less than or equal to 6 hours.

In some embodiments, a reaction temperature of reacting the halogen-containing compound with the first compound is 25 °C to 100 °C.

In some embodiments, a reaction time of reacting the halogen-containing compound with the first compound is 30 minutes to 60 minutes.

The present disclosure provides a method of preparing an ionic liquid, and the method includes the following steps. A halogen-containing compound is reacted with a first compound in a first microwave device to form a second compound, in which the halogen-containing compound includes a halohydrocarbon, a sulfonyl halide, or a combination thereof, the first compound includes an amine compound having a tertiary amine group, a phosphine compound, or a combination thereof, the second compound includes a first quaternary ammonium salt, a first quaternary phosphonium salt, or a combination thereof, and the amine compound having the tertiary amine group includes a structure as shown in formula (1-1), an imidazole compound, a pyrrolidine compound, a pyridine compound, a pyrrole compound, a pyrazole compound, a structure as shown in formula (1-2), an imidazoline compound, a pyrazoline compound, or combinations thereof, 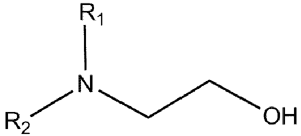 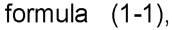 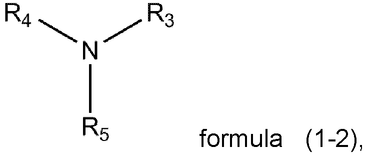 in which R₁ to R₅ are independently a C1 to C22 linear alkyl group, a C1 to C22 branched alkyl group, a C4 to C6 cycloalkyl group, or a C4 to C6 heterocycloalkyl group. The second compound is reacted with a lithium salt in a second microwave device to form a third compound, in which the third compound includes a second quaternary ammonium salt, a second quaternary phosphonium salt, or a combination thereof, and anions of the second compound and the third compound are different.

In some embodiments, a microwave power of reacting the halogen-containing compound with the first compound in the first microwave device and a microwave power of reacting the second compound with the lithium salt reacted in the second microwave device are respectively 700 watts to 1400 watts.

**In** some embodiments, a reaction pressure of reacting the halogen-containing compound with the first compound in the first microwave device and a reaction pressure of reacting the second compound with the lithium salt in the second microwave device are respectively greater than 1 atm and less than or equal to 2 atm.

**In** some embodiments, a reaction temperature of reacting the halogen-containing compound with the first compound in the first microwave device and a reaction temperature of reacting the second compound with the lithium salt in the second microwave device are respectively 60 °C to 250 °C.

**In** some embodiments, the anion of the second compound is a halide ion or a structure as shown in formula (4-1), and the anion of the third compound is bis(trifluoromethanesulfonyl)imide anion, bis(fluorosulfonyl)imide anion, tetrafluoroborate anion, hexafluorophosphate anion, trifluoromethanesulfonate anion, acetate ion, a structure as shown in formula (4-2), a structure as shown in formula (4-3), a structure as shown in formula (4-4), or combinations thereof, SO₂X₃⁻ formula (4-1), 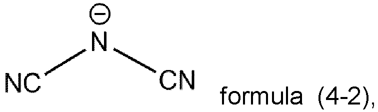 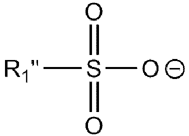 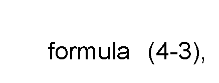 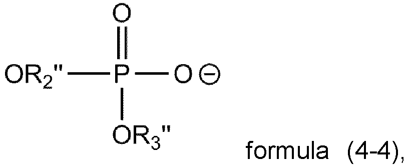 in which X₃ is fluorine, chlorine, bromine, or iodine, and R₁", R₂", and R₃" are independently a C1 to C22 linear alkyl group, a C1 to C22 branched alkyl group, a C4 to C6 cycloalkyl group, or a C4 to C6 heterocycloalkyl group.

In some embodiments, a molar ratio of the halogen-containing compound to the first compound is 1:1 to 2:1, and a molar ratio of the second compound to the lithium salt is 1:1 to 1:2.

In some embodiments, a reaction time of reacting the halogen-containing compound with the first compound in the first microwave device and a reaction time of reacting the second compound with the lithium salt in the second microwave device are independently 5 minutes to 30 minutes.

### BRIEF DESCRIPTION OF THE DRAWINGS

Figs. 1 and 2 are respectively flow diagrams of the methods of preparing the ionic liquid, in accordance with various embodiments of the present disclosure.
Fig. 3 shows Fourier transform infrared spectra of the ionic liquids, in accordance with various embodiments of the present disclosure.
Fig. 4 shows Raman spectra of the ionic liquids, in accordance with various embodiments of the present disclosure.
Figs. 5 and 6 respectively show linear sweep voltammetry curves of the ionic liquids, in accordance with various embodiments of the present disclosure.

### DETAILED DESCRIPTION

The present disclosure provides a method of preparing an ionic liquid, and the method prepares the ionic liquid in a microwave device, thereby reducing the time for preparing the ionic liquid. In addition, the ionic liquid with low moisture content, high ionic conductivity, low viscosity, and wide electrochemical stability window can be prepared through the preparation method of the present disclosure. It causes the ionic liquid prepared by the present disclosure more suitable as an electrolyte in batteries than the liquid electrolytes, the solid electrolytes, the special additives, the gelled electrolytes, or the flame retardants.

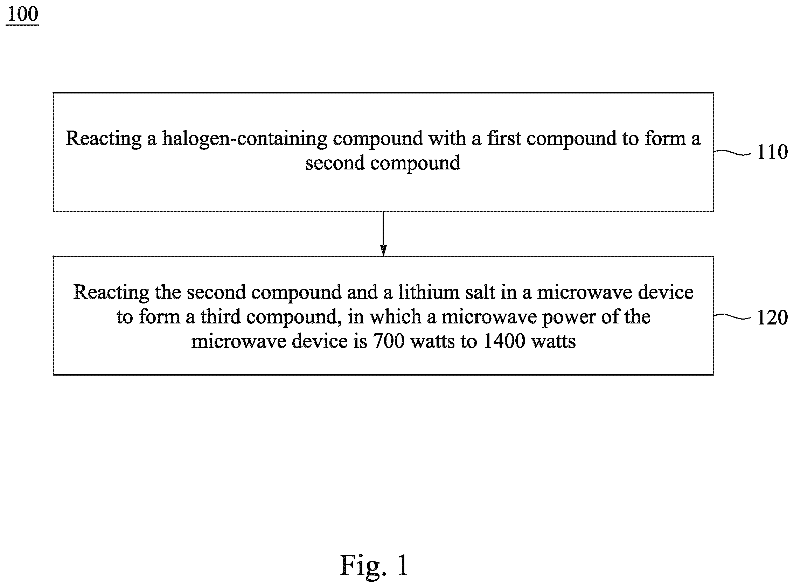

Fig. 1 is a flow diagram of a method 100 of preparing the ionic liquid, in accordance with various embodiments of the present disclosure. Refer to Fig. 1. The method 100 of preparing the ionic liquid includes steps 110 and 120. In the step 110, a halogen-containing compound is reacted with a first compound to form a second compound, in which the halogen-containing compound includes a halohydrocarbon, a sulfonyl halide, or a combination thereof, the first compound includes an amine compound having a tertiary amine group, a phosphine compound, or a combination thereof, and the second compound includes a first quaternary ammonium salt, a first quaternary phosphonium salt, or a combination thereof. In the step 120, the second compound is reacted with a lithium salt in a microwave device to form a third compound, in which the third compound includes a second quaternary ammonium salt, a second quaternary phosphonium salt, or a combination thereof, and anions of the second compound and the third compound are different. The third compound is the ionic liquid. A microwave power of the microwave device is 700 watts to 1400 watts, for example, 700, 750, 800, 850, 900, 950, 1000, 1050, 1100, 1150, 1200, 1250, 1300, 1350, or 1400 watts. When the microwave power is 700 watts to 1400 watts, a reaction time required to form the third compound can be shortened.

In some embodiments, the amine compound having the tertiary amine group includes a structure as shown in formula (1-1), an imidazole compound, a pyrrolidine compound, a pyridine compound, a pyrrole compound, a pyrazole compound, a structure as shown in formula (1-2), an imidazoline compound, a pyrazoline compound, or combinations thereof. The formula (1-1) and the formula (1-2) are shown as follows: 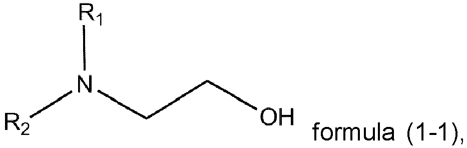 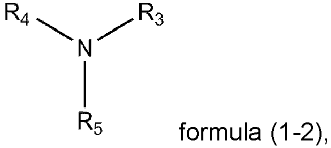 in which R₁ to R₅ are independently a C1 to C22 linear alkyl group, a C1 to C22 branched alkyl group, a C4 to C6 cycloalkyl group, or a C4 to C6 heterocycloalkyl group. The C1 to C22 linear alkyl group and the C1 to C22 branched alkyl group can have a carbon number of 1, 2, 4, 6, 8, 10, 12, 14, 16, 18, 20, or 22, and the C4 to C6 cycloalkyl group and the C4 to C6 heterocycloalkyl group can have a carbon number of 4, 5, or 6.

In some embodiments, the imidazole compound, the pyrrolidine compound, the pyridine compound, the pyrrole compound, the pyrazole compound, the imidazoline compound, and the pyrazoline compound are respectively structures as shown in formula (1-3), formula (1-4), formula (1-5), formula (1-6), formula (1-7), formula (1-8), and formula (1-9). The structures are respectively shown as follows: 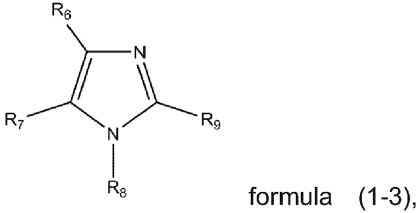 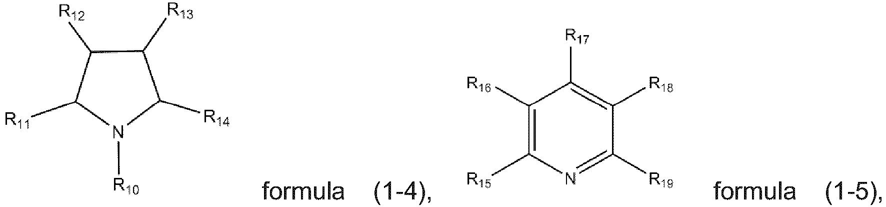 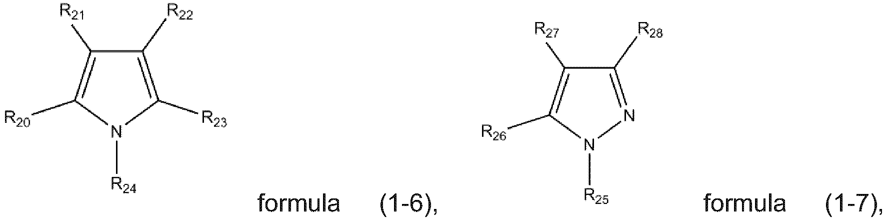 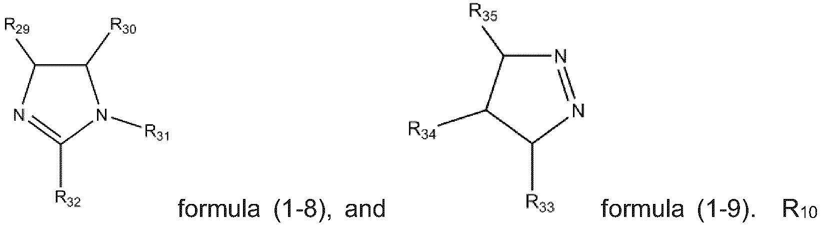 and R₂₄ are independently a C1 to C22 linear alkyl group, a C1 to C22 branched alkyl group, a C4 to C6 cycloalkyl group, or a C4 to C6 heterocycloalkyl group. The C1 to C22 linear alkyl group and the C1 to C22 branched alkyl group can have a carbon number of 1, 2, 4, 6, 8, 10, 12, 14, 16, 18, 20, or 22, and the C4 to C6 cycloalkyl group and the C4 to C6 heterocycloalkyl group can have a carbon number of 4, 5, or 6. R₆ to R₉, R₁₁ to R₂₃, R₂₅ to R₃₅ are independently hydrogen, a C1 to C22 linear alkyl group, a C1 to C22 branched alkyl group, a C4 to C6 cycloalkyl group, or a C4 to C6 heterocycloalkyl group. The C1 to C22 linear alkyl group and the C1 to C22 branched alkyl group can have a carbon number of 1, 2, 4, 6, 8, 10, 12, 14, 16, 18, 20, or 22, and the C4 to C6 cycloalkyl group and the C4 to C6 heterocycloalkyl group can have a carbon number of 4, 5, or 6.

In some embodiments, the phosphine compound has a structure as shown in formula (1-10): 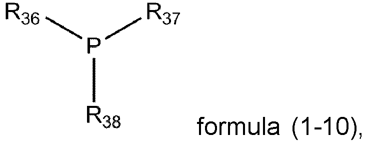 in which R₃₆ to R₃₈ are independently a C1 to C22 linear alkyl group, a C1 to C22 branched alkyl group, a C4 to C6 cycloalkyl group, or a C4 to C6 heterocycloalkyl group. The C1 to C22 linear alkyl group and the C1 to C22 branched alkyl group can have a carbon number of 1, 2, 4, 6, 8, 10, 12, 14, 16, 18, 20, or 22, and the C4 to C6 cycloalkyl group and the C4 to C6 heterocycloalkyl group can have a carbon number of 4, 5, or 6.

In some embodiments, the halohydrocarbon has a structure as shown in formula (2-1), and the sulfonyl halide has a structure as shown in formula (2-2):

R₁'-X₁ formula (2-1),

R₂'-SO₂X₂ formula (2-2),

in which R₁' and R₂' are independently a C1 to C22 linear alkyl group, a C1 to C22 branched alkyl group, a C4 to C6 cycloalkyl group, a C4 to C6 heterocycloalkyl group, or a C6 to C10 aryl group, and X₁ and X₂ are independently fluorine, chlorine, bromine, or iodine. The C1 to C22 linear alkyl group and the C1 to C22 branched alkyl group can have a carbon number of 1, 2, 4, 6, 8, 10, 12, 14, 16, 18, 20, or 22, the C4 to C6 cycloalkyl group and the C4 to C6 heterocycloalkyl group can have a carbon number of 4, 5, or 6, and the C6 to C10 aryl group can have a carbon number of 6, 7, 8, 9, or 10.

In some embodiments, a molar ratio of the halogen-containing compound to the first compound is 1:1 to 2:1, for example, 1:1, 1.2:1, 1.4:1, 1.6:1, 1.8:1, or 2:1. When the molar ratio of the halogen-containing compound to the first compound is 1:1 to 2:1, the first compound can be completely reacted to form the second compound. In some embodiments, a reaction temperature of reacting the halogen-containing compound with the first compound is 25 °C to 100 °C, for example, 25, 30, 35, 40, 45, 50, 55, 60, 65, 70, 75, 80, 85, 90, 95, or 100 °C. In some embodiments, a reaction time of reacting the halogen-containing compound with the first compound is 30 minutes to 60 minutes, for example, 30, 40, 50, or 60 minutes.

Please refer to Fig. 1 again. In some embodiments, before the step 110 is performed, the first compound is placed in a closed reaction container for stirring, and then the halogen-containing compound is added into the closed reaction container. In some embodiments, a temperature of placing the first compound in the closed reaction container for stirring is 10 °C to 100 °C, for example, 10, 20, 30, 40, 50, 60, 70, 80, 90, or 100 °C. In some embodiments, the closed reaction container is a reaction kettle. In some embodiments, the closed reaction container is filled with the inert gas, such as nitrogen gas, argon gas, helium gas, or neon gas. If the closed reaction container is filled with the inert gas, side reactions can be avoided.

Please refer to Fig. 1 again. More specifically, in the step 110, the halogen-containing compound undergoes an alkylation reaction with the first compound to form the second compound. The hydrocarbon group (for example, R₁' of the formula (2-1) and R₂' of the formula (2-2)) of the halogen-containing compound bonds with the tertiary amine or the tertiary phosphine of the first compound to form the second compound.

In some embodiments, the second compound having a 1-ethyl-3-methylimidazolium bromide (EMIM Br) structure is formed by reacting methyl bromide and the imidazole compound (the structure as shown in the formula (1-3), R₈ is an ethyl group, and R₆, R₇, and R₉ are hydrogen). In some embodiments, the second compound having a 1-butyl-1-methylpyrrolidinium bromide (Py14 Br) structure is formed by reacting butyl bromide and the pyrrolidine compound (the structure as shown in the formula (1-4), R₁₀ is a methyl group, and R₁₁ to R₁₄ are hydrogen). In some embodiments, the second compound having a structure as shown in formula (2-3) is formed by reacting butyl bromide and the pyridine compound (the structure as shown in the formula (1-5), R₁₇ is a methyl group, and R₁₅, R₁₆, R₁₈, and R₁₉ are hydrogen). In some embodiments, the second compound having a structure as shown in formula (2-4) is formed by reacting ethyl iodide and the structure as shown in the formula (1-2) (R₃, R₄, and R₅ are butyl groups). The formula (2-3) and the formula (2-4) are shown as follows:

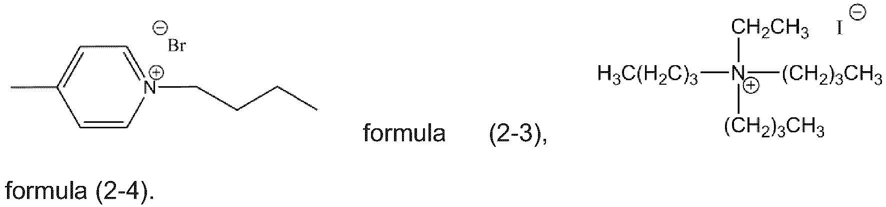

In some embodiments, the lithium salt includes lithium bis(trifluoromethanesulfonyl)imide (LiTFSI), lithium bis(fluorosulfonyl)imide (LiFSI), lithium tetrafluoroborate (LiBF₄), lithium hexafluorophosphate (LiPF₆), lithium trifluoromethanesulfonate (LiOTf), lithium acetate (CH₃COOLi), a structure as shown in formula (3-1), a structure as shown in formula (3-2), a structure as shown in formula (3-3), or combinations thereof. The formula (3-1), the formula (3-2), and the formula (3-3) are shown as follows: 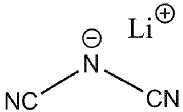 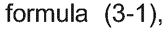 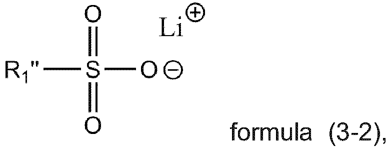 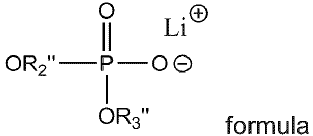 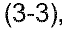 in which R₁", R₂", and R₃" are independently a C1 to C22 linear alkyl group, a C1 to C22 branched alkyl group, a C4 to C6 cycloalkyl group, or a C4 to C6 heterocycloalkyl group. The C1 to C22 linear alkyl group and the C1 to C22 branched alkyl group can have a carbon number of 1, 2, 4, 6, 8, 10, 12, 14, 16, 18, 20, or 22, and the C4 to C6 cycloalkyl group and the C4 to C6 heterocycloalkyl group can have a carbon number of 4, 5, or 6.

In some embodiments, the anion of the second compound includes a halide ion or a structure as shown in formula (4-1), the halide ion includes fluoride ion, chloride ion, bromide ion, or iodide ion, and the anion of the third compound includes bis(trifluoromethanesulfonyl)imide anion (TFSI⁻), bis(fluorosulfonyl)imide anion (FSI⁻), tetrafluoroborate anion (BF₄⁻), hexafluorophosphate anion (PF₆⁻), trifluoromethanesulfonate anion (OTf⁻), acetate ion (CH₃COO⁻), a structure as shown in formula (4-2), a structure as shown in formula (4-3), a structure as shown in formula (4-4), or combinations thereof. The formula (4-1), the formula (4-2), the formula (4-3), and the formula (4-4) are shown as follows: SO₂X₃⁻ formula (4-1), 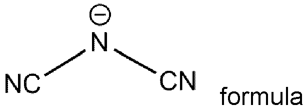 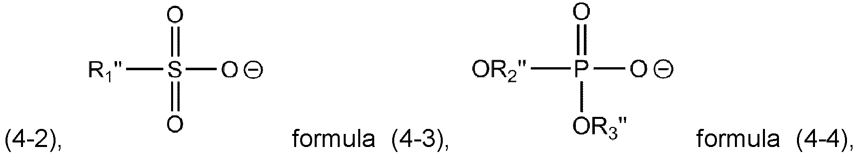 in which X₃ is fluorine, chlorine, bromine, or iodine, and R₁", R₂", and R₃" are independently a C1 to C22 linear alkyl group, a C1 to C22 branched alkyl group, a C4 to C6 cycloalkyl group, or a C4 to C6 heterocycloalkyl group. The C1 to C22 linear alkyl group and the C1 to C22 branched alkyl group can have a carbon number of 1, 2, 4, 6, 8, 10, 12, 14, 16, 18, 20, or 22, and the C4 to C6 cycloalkyl group and the C4 to C6 heterocycloalkyl group can have a carbon number of 4, 5, or 6.

In some embodiments, a molar ratio of the second compound to the lithium salt is 1:1 to 1:2, for example, 1:1, 1:1.2, 1:1.4, 1:1.6, 1:1.8, or 1:2. When the molar ratio of the second compound to the lithium salt is 1:1 to 1:2, the second compound can be completely reacted to form the third compound.

In some embodiments, a reaction pressure of reacting the second compound with the lithium salt in the microwave device is greater than 1 atm and less than or equal to 2 atm, for example, 1.1, 1.2, 1.3, 1.4, 1.5, 1.6, 1.7, 1.8, 1.9, or 2 atm. When the reaction pressure of reacting the second compound with the lithium salt in the microwave device is greater than 1 atm and less than or equal to 2 atm, the reaction time required to form the third compound can be shortened. In some embodiments, the microwave device is a reaction kettle having a microwave function. In some embodiments, a reaction temperature of reacting the second compound with the lithium salt in the microwave device is 60 °C to 250 °C, for example, 60, 70, 80, 90, 100, 110, 120, 130, 140, 150, 160, 170, 180, 190, 200, 210, 220, 230, 240, or 250 °C. The reactivity is improved when the reaction temperature of reacting the second compound with the lithium salt in the microwave device is 60 °C to 250 °C. In some embodiments, a reaction time of reacting the second compound with the lithium salt in the microwave device is 5 minutes to 30 minutes, for example, 5, 10, 15, 20, 25, or 30 minutes. The pressure of the microwave device will not be excessively high and the reactivity is improved when the reaction time of reacting the second compound with the lithium salt in the microwave device is 5 minutes to 30 minutes.

Please refer to Fig. 1 again. In some embodiments, before the step 120 is performed, the second compound is dissolved in the first solvent, after that, the second compound dissolved in the first solvent is placed in a reaction container. Then, the lithium salt is dissolved in the second solvent, and the lithium salt dissolved in the second solvent is added into the reaction container and mixed with the second compound. In some embodiments, the first solvent and the second solvent are environmentally friendly polar solvents, such as water, methanol, ethanol, acetone, or combinations thereof. When the first solvent and the second solvent are respectively water, methanol, ethanol, acetone, or combinations thereof, the formed third compound and the by-product(s) are easy to separate after the second compound is reacted with the lithium salt. In some embodiments, the reaction container is a microwave device. In some embodiments, a mixing temperature of mixing the lithium salt dissolved in the solvent with the second compound is 25 °C to 100 °C, for example, 25, 30, 35, 40, 45, 50, 55, 60, 65, 70, 75, 80, 85, 90, 95, or 100 °C. A mixing time of mixing the lithium salt dissolved in the solvent with the second compound is 15 minutes to 30 minutes, such as 15, 20, 25, or 30 minutes. When the mixing time falls within the range, the lithium salt dissolved in the solvent can mix rapidly and evenly with the second compound.

Please refer to Fig. 1 again. In some embodiments, the method 100 of preparing the ionic liquid further includes after reacting the second compound with the lithium salt in the microwave device, performing a heating operation, in which a heating temperature of the heating operation is 60 °C to 180 °C, for example, 60, 70, 80, 90, 100, 110, 120, 130, 140, 150, 160, 170, or 180 °C. In some embodiments, a heating time of the heating operation is less than or equal to 6 hours, for example, 0.5, 1, 1.5, 2, 2.5, 3, 3.5, 4, 4.5, 5, 5.5, or 6 hour(s). When the heating temperature of the heating operation is 60 °C to 180 °C, the reactivity of the second compound and the lithium salt is enhanced, and it is less likely to produce by-products.

Please refer to Fig. 1 again. In some embodiments, after the step 120 is performed, the third compound and the by-product(s) are separated, and the third compound is purified through a third solvent. In some embodiments, after the step 120 is performed, the third compound is not dissolved in the aforementioned first solvent and the second solvent to form a non-aqueous phase layer, and the by-product(s) is/are dissolved in the first solvent and the second solvent to form an aqueous phase layer. In some embodiments, the third solvent includes water, methanol, ethanol, acetone, or combinations thereof.

Please refer to Fig. 1 again. In some embodiments, after the step 120 is performed, the third compound is mixed with a fourth solvent, and then the mixture of the third compound and the fourth solvent is concentrated under reduced pressure to form a high purity third compound. In some embodiments, the fourth solvent includes a high volatility and an environmentally friendly solvent, such as methanol, ethanol, acetone, or combinations thereof. In some embodiments, a weight ratio of the third compound to the fourth solvent is 3:1 to 19:1, for example, 3:1, 5:1, 7:1, 9:1, 11:1, 13:1, 15:1, 17:1, or 19:1. In some embodiments, a temperature of concentration under reduced pressure is 30 °C to 100 °C, for example, 30, 40, 50, 60, 70, 80, 90, or 100 °C. In some embodiments, a time of concentration under reduced pressure is 10 minutes to 60 minutes, for example, 10, 20, 30, 40, 50, or 60 minutes. When the third compound is mixed with the fourth solvent, it is helpful to reduce the time of concentration under reduced pressure. When the third compound is mixed with the fourth solvent, and then the mixture is concentrated under reduced pressure, the high purity third compound has low moisture content.

Please refer to Fig. 1 again. More specifically, in the step 120, the second compound undergoes an anion replacement reaction with the lithium salt to form the third compound, in which the anion of the second compound is replaced by the anion of the lithium salt to form the third compound.

In some embodiments, the third compound having a 1-ethyl-3-methylimidazolium bis(trifluoromethanesulfonyl)imide (EMIM TFSI) structure is formed by reacting the second compound having the EMIM Br structure with LiTFSI (lithium salt). In some embodiments, the third compound having a 1-ethyl-3-methylimidazolium bis(fluorosulfonyl)imide (EMIM FSI) structure is formed by reacting the second compound having the EMIM Br structure with LiFSI (lithium salt). In some embodiments, the third compound having a 1-butyl-1-methylpyrrolidinium bis(trifluoromethanesulfonyl)imide (Py14 TFSI) structure is formed by reacting the second compound having the Py14 Br structure with LiTFSI (lithium salt).

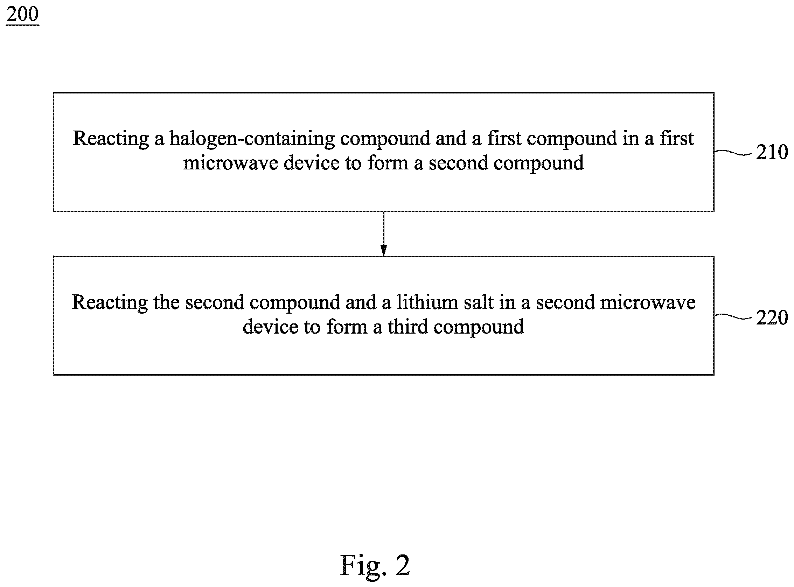

Fig. 2 is a flow diagram of the method 200 of preparing the ionic liquid, in accordance with various embodiments of the present disclosure. Refer to Fig. 2. The method 200 of preparing the ionic liquid includes steps 210 and 220. In the step 210, a halogen-containing compound and a first compound are reacted in a first microwave device to form a second compound, in which the halogen-containing compound includes a halohydrocarbon, a sulfonyl halide, or a combination thereof, the first compound includes an amine compound having a tertiary amine group, a phosphine compound, or a combination thereof, the second compound includes a first quaternary ammonium salt, a first quaternary phosphonium salt, or a combination thereof, and the amine compound having the tertiary amine group includes a structure as shown in formula (1-1), an imidazole compound, a pyrrolidine compound, a pyridine compound, a pyrrole compound, a pyrazole compound, a structure as shown in formula (1-2), an imidazoline compound, a pyrazoline compound, or combinations thereof. The formula (1-1) and the formula (1-2) are shown as follows: 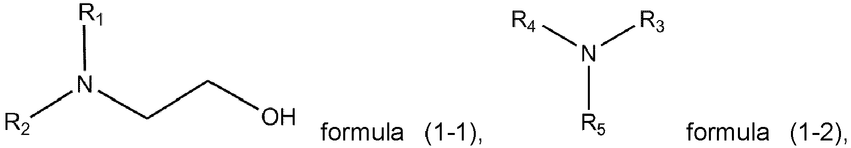 in which R₁ to R₅ are independently a C1 to C22 linear alkyl group, a C1 to C22 branched alkyl group, a C4 to C6 cycloalkyl group, or a C4 to C6 heterocycloalkyl group. The C1 to C22 linear alkyl group and the C1 to C22 branched alkyl group can have a carbon number of 1, 2, 4, 6, 8, 10, 12, 14, 16, 18, 20, or 22, and the C4 to C6 cycloalkyl group and the C4 to C6 heterocycloalkyl group can have a carbon number of 4, 5, or 6. In the step 220, the second compound is reacted with a lithium salt in a second microwave device to form a third compound, in which the third compound includes a second quaternary ammonium salt, a second quaternary phosphonium salt, or a combination thereof, and anions of the second compound and the third compound are different. The third compound is the ionic liquid. When the halogen-containing compound is reacted with the first compound in the first microwave device, a reaction time required to form the second compound can be shortened. When the second compound is reacted with the lithium salt in the second microwave device, a reaction time required to form the third compound can be shortened. In some embodiments, the first microwave device and the second microwave device are the same devices. In some embodiments, the first microwave device and the second microwave device are different devices.

In some embodiments, refer to the aforementioned structures as shown in the formula (1-3), formula (1-4), formula (1-5), formula (1-6), formula (1-7), formula (1-8), and formula (1-9) for the imidazole compound, the pyrrolidine compound, the pyridine compound, the pyrrole compound, the pyrazole compound, the imidazoline compound, and the pyrazoline compound respectively. In some embodiments, refer to the aforementioned structure as shown in the formula (1-10) for the phosphine compound. In some embodiments, refer to the aforementioned structure as shown in the formula (2-1) and formula (2-2) for the halohydrocarbon and the sulfonyl halide respectively. In some embodiments, refer to the lithium salt used in the step 120 for a lithium salt used in the step 220.

In some embodiments, a microwave power of reacting the halogen-containing compound with the first compound in the first microwave device and a microwave power of reacting the second compound with the lithium salt reacted in the second microwave device are respectively 700 watts to 1400 watts, for example, 700, 750, 800, 850, 900, 950, 1000, 1050, 1100, 1150, 1200, 1250, 1300, 1350, or 1400 watts. When the microwave power of reacting the halogen-containing compound with the first compound in the first microwave device is 700 watts to 1400 watts, a reaction time required to form the second compound can be shortened. When the microwave power of reacting the second compound with the lithium salt reacted in the second microwave device is 700 watts to 1400 watts, a reaction time required to form the third compound can be shortened.

In some embodiments, a reaction pressure of reacting the halogen-containing compound with the first compound in the first microwave device and a reaction pressure of reacting the second compound with the lithium salt in the second microwave device are respectively greater than 1 atm and less than or equal to 2 atm, for example, 1.1, 1.2, 1.3, 1.4, 1.5, 1.6, 1.7, 1.8, 1.9, or 2 atm. When the reaction pressure of reacting the halogen-containing compound with the first compound in the first microwave device is greater than 1 atm and less than or equal to 2 atm, the reaction time required to form the second compound can be shortened. When the reaction pressure of reacting the second compound with the lithium salt in the second microwave device is greater than 1 atm and less than or equal to 2 atm, the reaction time required to form the third compound can be shortened. In some embodiments, the first microwave and the second microwave are reaction kettles with a microwave function.

In some embodiments, a reaction temperature of reacting the halogen-containing compound with the first compound in the first microwave device and a reaction temperature of reacting the second compound with the lithium salt in the second microwave device are respectively 60 °C to 250 °C, for example, 60, 70, 80, 90, 100, 110, 120, 130, 140, 150, 160, 170, 180, 190, 200, 210, 220, 230, 240, or 250 °C. When the reaction temperature of reacting the halogen-containing compound with the first compound in the first microwave device and the reaction temperature of reacting the second compound with the lithium salt in the second microwave device are respectively 60 °C to 250 °C, the reactivity is improved. In some embodiments, a reaction time of reacting the halogen-containing compound with the first compound in the first microwave device and a reaction time of reacting the second compound with the lithium salt in the second microwave device are respectively 5 minutes to 30 minutes, for example, 5, 10, 15, 20, 25, or 30 minutes. When the reaction time of reacting the halogen-containing compound with the first compound in the first microwave device is 5 minutes to 30 minutes, the pressure of the first microwave device will not be excessively high and the reactivity is improved. When the reaction time of reacting the second compound with the lithium salt in the second microwave device is 5 minutes to 30 minutes, the pressure of the second microwave device will not be excessively high and the reactivity is improved.

In some embodiments, the anion of the second compound is a halide ion or a structure as shown in formula (4-1), the halide ion includes fluoride ion, chloride ion, bromide ion, or iodide ion, and the anion of the third compound is bis(trifluoromethanesulfonyl)imide anion (TFSI⁻), bis(fluorosulfonyl)imide anion (FSI⁻), tetrafluoroborate anion (BF₄⁻), hexafluorophosphate anion (PF₆⁻), trifluoromethanesulfonate anion (OTf⁻), acetate ion (CH₃COO⁻), a structure as shown in formula (4-2), a structure as shown in formula (4-3), a structure as shown in formula (4-4), or combinations thereof. The formula (4-1), the formula (4-2), the formula (4-3), and the formula (4-4) are shown as follows: SO₂X₃⁻formula (4-1), 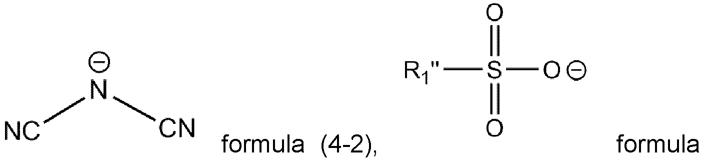 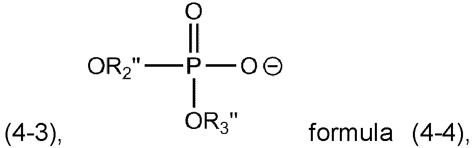 in which X₃ is fluorine, chlorine, bromine, or iodine, and R₁", R₂", and R₃" are independently a C1 to C22 linear alkyl group, a C1 to C22 branched alkyl group, a C4 to C6 cycloalkyl group, or a C4 to C6 heterocycloalkyl group. The C1 to C22 linear alkyl group and the C1 to C22 branched alkyl group can have a carbon number of 1, 2, 4, 6, 8, 10, 12, 14, 16, 18, 20, or 22, and the C4 to C6 cycloalkyl group and the C4 to C6 heterocycloalkyl group can have a carbon number of 4, 5, or 6.

In some embodiments, a molar ratio of the halogen-containing compound to the first compound is 1:1 to 2:1, for example, 1:1, 1.2:1, 1.4:1, 1.6:1, 1.8:1, or 2:1, and a molar ratio of the second compound to the lithium salt is 1:1 to 1:2, for example, 1:1, 1:1.2, 1:1.4, 1:1.6, 1:1.8, or 1:2. When the molar ratio of the halogen-containing compound to the first compound is 1:1 to 2:1, the first compound can be completely reacted to form the second compound. When the molar ratio of the second compound to the lithium salt is 1:1 to 1:2, the second compound can be completely reacted to form the third compound.

Please refer to Fig. 2 again. In some embodiments, before the step 210 is performed, the halogen-containing compound and the first compound are placed in a closed reaction container for stirring. In some embodiments, a temperature of placing the first compound in the closed reaction container for stirring is 10 °C to 100 °C, for example, 10, 20, 30, 40, 50, 60, 70, 80, 90, or 100 °C. In some embodiments, the closed reaction container is a reaction kettle.

Please refer to Fig. 2 again. In some embodiments, before the step 210 is performed, a heating operation is performed, in which a heating temperature of the heating operation is 60 °C to 180 °C, for example, 60, 70, 80, 90, 100, 110, 120, 130, 140, 150, 160, 170, or 180 °C. In some embodiments, a heating time of the heating operation is less than or equal to 6 hours, for example, 0.5, 1, 1.5, 2, 2.5, 3, 3.5, 4, 4.5, 5, 5.5, or 6 hour(s). When the heating temperature of the heating operation is 60 °C to 180 °C, the reactivity of the second compound and the lithium salt is enhanced and it is less likely to produce by-products. In some embodiments, refer to the embodiments and the advantages corresponding to these embodiments before and after performing the step 120 for the other embodiments and the advantages corresponding to these embodiments before and after performing the step 220 respectively.

Please refer to Fig. 2 again. More specifically, in the step 210, the halogen-containing compound undergoes an alkylation reaction with the first compound to form the second compound. Hydrocarbon group (for example, R₁' of the formula (2-1) and R₂' of the formula (2-2)) of the halogen-containing compound bonds with the tertiary amine or the tertiary phosphine of the first compound to form the second compound. In the step 220, the second compound undergoes an anion replacement reaction with the lithium salt to form the third compound. The anion of the second compound is replaced by the anion of the lithium salt to form the third compound. In some embodiments, refer to the second compound and the third compound formed in the steps 110 and 120 for the structures of the second compound and the third compound formed in the steps 210 and 220 respectively.

### Experimental Example 1: preparation of ionic liquids

In example 1-1, methyl bromide and the imidazole compound (the structure as shown in formula (1-3), R₆, R₇, and R₉ are hydrogen, and R₈ is an ethyl group) at a molar ratio of 1.05:1 were reacted at 60 °C for 30 minutes to form EMIM Br. EMIM Br and the lithium salt (LiTFSI and LiFSI were mixed at a weight ratio of 8.5:1.5) at a molar ratio of 1:1.1 were respectively dissolved in the water, and then mixed and stirred at 60 °C for 15 minutes to form a mixture. After that, the mixture was reacted at 1000 watts for 30 minutes, and then heated at 150 °C for 6 hours to form EMIM TFSI/FSI.

In example 1-2, EMIM TFSI/FSI of the example 1-1 was mixed with methanol at a weight ratio of 95:5, and then concentrated under reduced pressure at 30 °C for 30 minutes to form high purity EMIM TFSI/FSI.

In example 1-3, methyl bromide and the imidazole compound (the structure as shown in formula (1-3), R₆, R₇, and R₉ are hydrogen, and R₈ is an ethyl group) at a molar ratio of 1.05:1 were reacted at 60 °C for 30 minutes to form EMIM Br. EMIM Br and LiTFSI at a molar ratio of 1:1.1 were respectively dissolved in the water, and then mixed and stirred at 60 °C for 15 minutes to form a mixture. After that, the mixture was reacted at 1000 watts for 30 minutes, and then heated at 150 °C for 6 hours to form EMIM TFSI. EMIM TFSI was mixed with methanol at a weight ratio of 95:5, and then concentrated under reduced pressure at 30 °C for 30 minutes to form high purity EMIM TFSI.

In example 1-4, butyl bromide and the pyrrolidine compound (the structure as shown in formula (1-4), R₁₀ is a methyl group, and R₁₁ to R₁₄ are hydrogen) at a molar ratio of 1.05:1 were reacted at 60 °C for 30 minutes to form Py14 Br. Py14 Br and LiTFSI at a molar ratio of 1:1.1 were respectively dissolved in the water, and then mixed and stirred at 60 °C for 15 minutes to form a mixture. After that, the mixture was reacted at 1000 watts for 30 minutes, and then heated at 150 °C for 6 hours to form Py14 TFSI. Py14 TFSI was mixed with methanol at a weight ratio of 95:5, and then concentrated under reduced pressure at 30 °C for 30 minutes to form high purity Py14 TFSI.

### Experimental Example 2: measurement of Fourier-transform infrared (FTIR) spectra and Raman spectra

In comparative example 2-1, commercially available ionic liquid EMIM TFSI (product name: 1-Ethyl-3-methylimidazolium bis(trifluoromethanesulfonyl)imide, manufacturer: IOLITEC) and commercially available ionic liquid EMIM FSI (product name: 1-Ethyl-3-methylimidazolium bis(fluorosulfonyl)imide, manufacturer: IOLITEC) were mixed and then formed ionic liquid EMIM TFSI/FSI, in which a weight ratio of commercially available ionic liquid EMIM TFSI to commercially available ionic liquid EMIM FSI was 8.5:1.5.

EMIM TFSI/FSI of the example 1-1, high purity EMIM TFSI/FSI of the example 1-2, and ionic liquid EMIM TFSI/FSI of the comparative example 2-1 were performed the measurements of the FTIR and the Raman spectra sequentially.

The measurement method and the parameter settings of the FTIR spectra: 50 microliters of EMIM TFSI/FSI of the example 1-1, high purity EMIM TFSI/FSI of the example 1-2, and ionic liquid EMIM TFSI/FSI of the comparative example 2-1 were dropped on a total reflection window of an attenuated total reflection (ATR) FTIR of a diamond substrate sequentially. The number of integrations was 16, the resolution level was 4, and the measurement was performed in ATR mode.

The measurement method and the parameter settings of the Raman spectra: 50 microliters of EMIM TFSI/FSI of the example 1-1, high purity EMIM TFSI/FSI of the example 1-2, and ionic liquid EMIM TFSI/FSI of the comparative example 2-1 were dropped on a glass substrate sequentially. The light wavelength of the light was 532 nm. The intensity was 9 millwatts. It was equipped with an aperture of 50 µm. The objective lens magnification was 50x magnification. The integration scan time was 2 seconds and the scan was repeated 150 times.

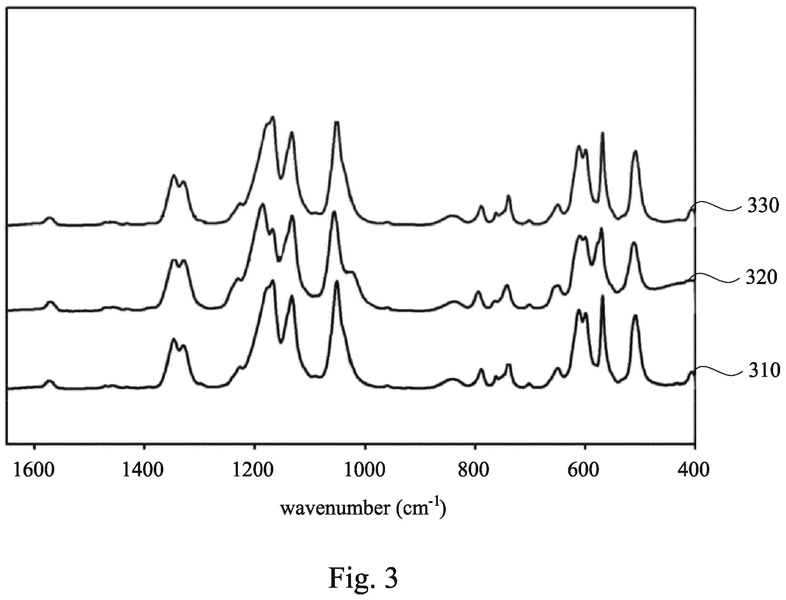

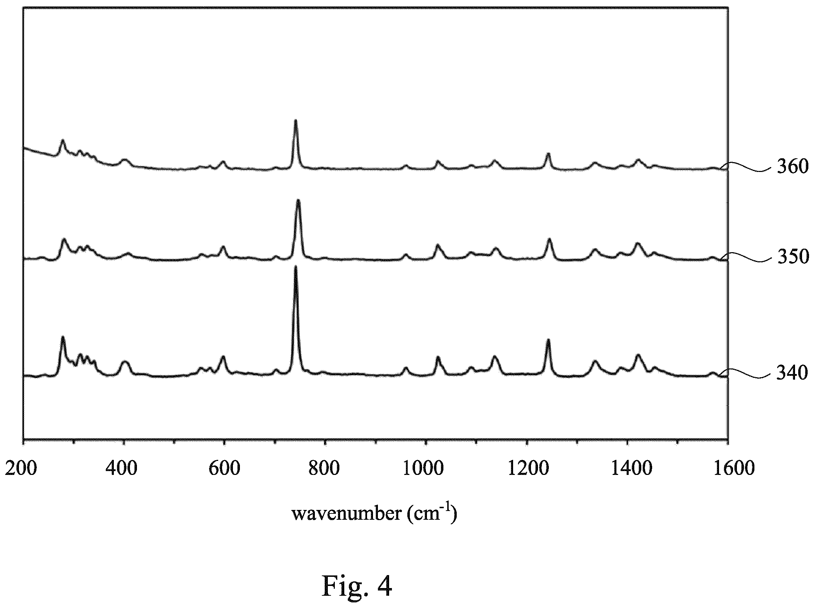

Fig. 3 shows Fourier transform infrared (FTIR) spectra of the ionic liquids, in accordance with various embodiments of the present disclosure. The curve 310 is the FTIR spectrum of the example 1-1, the curve 320 is the FTIR spectrum of the example 1-2, and the curve 330 is the FTIR spectrum of the comparative example 2-1. As shown in Fig. 3, there are essentially no differences in the FTIR spectra of the curves 310 to 330. It can be concluded that the structures of ionic liquid EMIM TFSI/FSI (curve 310) of the present disclosure, high purity ionic liquid EMIM TFSI/FSI (curve 320) of the present disclosure, and ionic liquid EMIM TFSI/FSI (curve 330) are the same. Fig. 4 shows Raman spectra of the ionic liquids, in accordance with various embodiments of the present disclosure. The curve 340 is the Raman spectrum of the example 1-1, the curve 350 is the Raman spectrum of the example 1-2, and the curve 360 is the Raman spectrum of the comparative example 2-1. As shown in Fig.4, there are essentially no differences in the Raman spectra of the curves 340 to 360. It can be concluded that the structures of prepared ionic liquid EMIM TFSI/FSI (the curve 340), high purity ionic liquid EMIM TFSI/FSI (the curve 350), and ionic liquid EMIM TFSI/FSI (the curve 360) are the same. Therefore, as illustrated by the FTIR and the Raman spectra, the method of preparing the ionic liquids of the present disclosure can successfully prepare the structures identical to that of the mixed commercially available ionic liquid.

### Experimental Example 3: measurement of conductivity, moisture content, and viscosity of ionic liquids

The commercially available ionic liquid used in the Experimental Example 3 was EMIM TFSI (product name: 1-Ethyl-3-methylimidazolium bis(trifluoromethanesulfonyl)imide, manufacturer: IOLITEC).

Example 3-1 and comparative example 3-1 were respectively dropped 50 microliters high purity EMIM TFSI of the example 1-3 and commercially available ionic liquid EMIM TFSI in separators, and then assembled with two stainless steel caps to form button cell batteries.

High purity EMIM TFSI of the example 1-3, commercially available ionic liquid EMIM TFSI, the button cell battery of the example 3-1, and the button cell battery of the comparative example 3-1 were performed the measurements of the conductivity sequentially. The measurement method of the conductivity is as follows: the probes of the conductivity meters were respectively immersed in high purity EMIM TFSI of the example 1-3 and commercially available ionic liquid EMIM TFSI, and then the ionic liquids were measured. The button cell batteries of the example 3-1 and the comparative example 3-1 were measured through the potentiostat/galvanostat.

**Table 1: the measurements of the conductivities**

| | Conductivity (S/cm) |
|---|---|
| example 1-3 | 8.3x10⁻³ |
| example 3-1 | 1.2x10⁻³ |
| commercially available ionic liquid EMIM TFSI | 8x10⁻³ |
| comparative example 3-1 | 1x10⁻³ |

Refer to table 1. The conductivities of EMIM TFSI of the example 1-3 and the button cell battery of the example 3-1 are respectively greater than the conductivities of commercially available ionic liquid EMIM TFSI and the button cell battery of the comparative example 3-1. It can be concluded that high purity EMIM TFSI (example 1-3) prepared by the present disclosure and the button cell battery (example 3-1) prepared by EMIM TFSI have a higher ionic conductivity than commercially available ionic liquid EMIM TFSI and the button cell battery (comparative example 3-1) of commercially available ionic liquid EMIM TFSI. Therefore, as illustrated in table 1, the method of preparing the ionic liquids of the present disclosure can prepare a higher ionic conductivity than the commercially available ionic liquid.

High purity EMIM TFSI of the example 1-3 and commercially available ionic liquid EMIM TFSI were performed the measurements of the moisture content sequentially. The measurement method of the moisture content is as follows: 1 milliliter of high purity EMIM TFSI of the example 1-3 and commercially available ionic liquid EMIM TFSI were respectively injected into the titration cell of the coulometric moisture titrator for measuring.

**Table 2: the measurements of moisture contents**

| | moisture content (ppm) |
|---|---|
| example 1-3 | 93 |
| commercially available ionic liquid EMIM TFSI | 167 |

Refer to table 2. The moisture content of EMIM TFSI of the example 1-3 is less than the moisture content of commercially available ionic liquid EMIM TFSI. It can be concluded that high purity EMIM TFSI (example 1-3) prepared by the present disclosure has a lower moisture content than commercially available ionic liquid EMIM TFSI. Therefore, as illustrated in table 2, the method of preparing the ionic liquids of the present disclosure can prepare a lower moisture content than the commercially available ionic liquid.

High purity ionic liquid of the example 1-3 and commercially available ionic liquid were performed the measurements of the viscosity sequentially. The measurement method of the viscosity is as follows: 1 milliliter of high purity EMIM TFSI of the example 1-3 and commercially available ionic liquid EMIM TFSI were respectively fill in the sample injection syringe of the viscometer for measuring.

**Table 3: the measurements of the viscosity**

| | viscosity (cP, 25 °C) |
|---|---|
| example 1-3 | 93 |
| commercially available ionic liquid EMIM TFSI | 167 |

Refer to table 3. The viscosity of high purity ionic liquid EMIM TFSI of the example 1-3 is less than the viscosity of commercially available ionic liquid EMIM TFSI. It can be concluded that high purity EMIM TFSI (example 1-3) prepared by the present disclosure has a lower viscosity than commercially available ionic liquid EMIM TFSI. Therefore, as illustrated in table 3, the method of preparing the ionic liquids of the present disclosure can prepare a lower viscosity than the commercially available ionic liquid.

### Experimental Example 4: measurements of linear sweep voltammetry (LSV) graphs of ionic liquids

High purity EMIM TFSI of the example 1-3 and high purity Py14 TFSI of the example 1-4 were performed the measurements of the LSV graphs sequentially.

The measurement method and the parameter settings of the LSV graphs: 50 microliters of high purity EMIM TFSI of the example 1-3 and high purity Py14 TFSI of the example 1-4 were dropped on separators respectively, and assembled with two stainless steel caps to form button cell batteries for measuring. The scan rate is 5 mV/s.

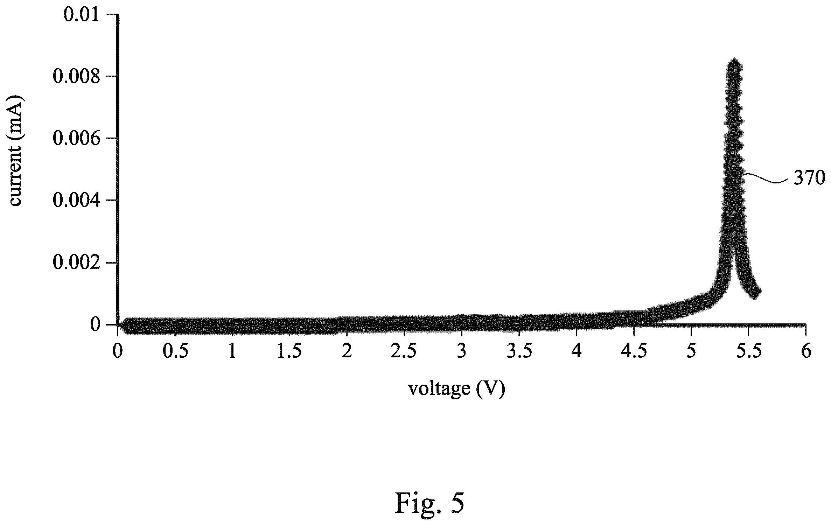

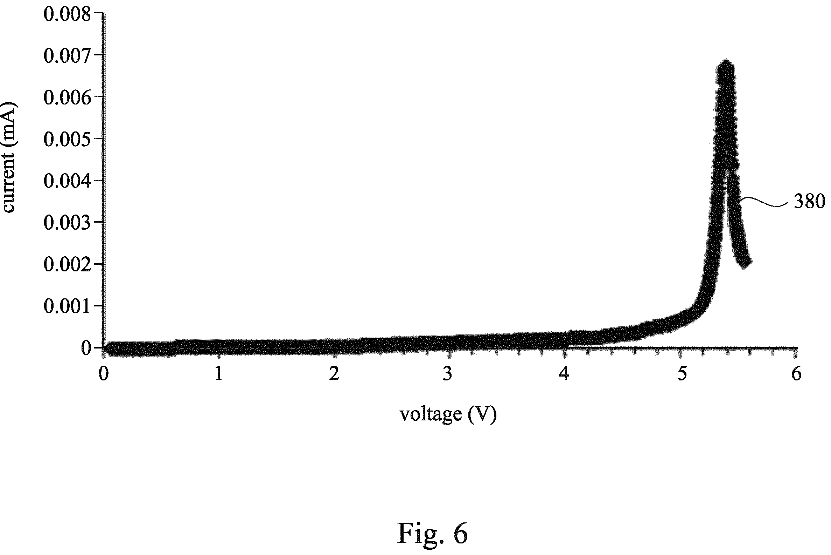

Figs. 5 and 6 show linear sweep voltammetry (LSV) curves of the ionic liquids, in accordance with various embodiments of the present disclosure. In Fig. 5, the curve 370 is a LSV curve of the example 1-3. In Fig. 6, the curve 380 is a LSV curve of the example 1-4. The measurements of the LSV graphs were using stainless steel as a working electrode and using lithium metal as a counter electrode and a reference electrode. Refer to Figs. 5 and 6. In the curves 370 and 380, there is almost no current appears when the voltage is less than 5 volts (V), and the current appears until 5 V to 5.5 V. Thus, the high purity ionic liquids (curves 370 and 380) prepared by the present disclosure have electrochemical stability windows greater than 5V.

In summary, the present disclosure provides the method of preparing the ionic liquid. Specifically, the method of the present disclosure includes preparing the ionic liquid in the microwave device to shorten the reaction time effectively. In some embodiments, 700 watts to 1400 watts of the microwave power used in the microwave device and/or the reaction pressure in the microwave device greater than 1 atm and less than or equal to 2 atm can shorten the preparation time of the ionic liquid. In some embodiments, after the ionic liquid is concentrated under reduced pressure, the moisture content of the ionic liquid decreases. Besides, the ionic liquid prepared by the method of the present disclosure has the properties of low moisture content, high ionic conductivity, low viscosity, electrochemical stability window greater than 5 V, and so on. Therefore, the ionic liquid prepared by the method of the present disclosure is applicable to the battery field.

## Claims

1. A method of preparing an ionic liquid, **characterized by** comprising:
reacting a halogen-containing compound with a first compound to form a second compound, wherein the halogen-containing compound comprises a halohydrocarbon, a sulfonyl halide, or a combination thereof, the first compound comprises an amine compound having a tertiary amine group, a phosphine compound, or a combination thereof, and the second compound comprises a first quaternary ammonium salt, a first quaternary phosphonium salt, or a combination thereof; and
reacting the second compound with a lithium salt in a microwave device to form a third compound, wherein the third compound comprises a second quaternary ammonium salt, a second quaternary phosphonium salt, or a combination thereof, anions of the second compound and the third compound are different, and a microwave power of the microwave device is 700 watts to 1400 watts.

2. The method of preparing the ionic liquid of claim 1, **characterized in that** the amine compound having the tertiary amine group comprises a structure as shown in formula (1-1), an imidazole compound, a pyrrolidine compound, a pyridine compound, a pyrrole compound, a pyrazole compound, a structure as shown in formula (1-2), an imidazoline compound, a pyrazoline compound, or combinations thereof, 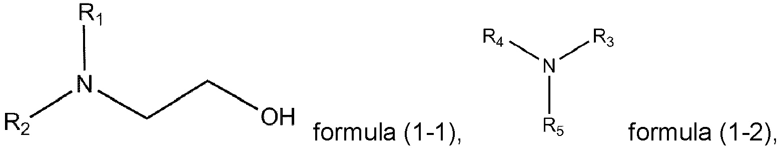 wherein R₁ to R₅ are independently a C1 to C22 linear alkyl group, a C1 to C22 branched alkyl group, a C4 to C6 cycloalkyl group, or a C4 to C6 heterocycloalkyl group.

3. The method of preparing the ionic liquid of claim 2, **characterized in that** the imidazole compound has a structure as shown in formula (1-3), 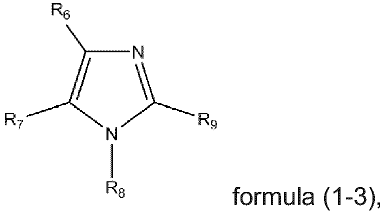 wherein R₆ to R₉ are independently hydrogen, a C1 to C22 linear alkyl group, a C1 to C22 branched alkyl group, a C4 to C6 cycloalkyl group, or a C4 to C6 heterocycloalkyl group.

4. The method of preparing the ionic liquid of claims 2 or 3, **characterized in that** the pyrrolidine compound has a structure as shown in formula (1-4), 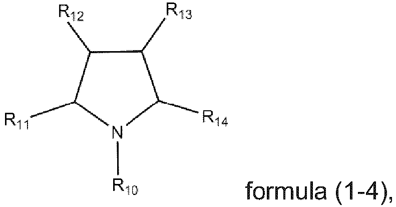 wherein R₁₀ is a C1 to C22 linear alkyl group, a C1 to C22 branched alkyl group, a C4 to C6 cycloalkyl group, or a C4 to C6 heterocycloalkyl group, and R₁₁ to R₁₄ are independently hydrogen, a C1 to C22 linear alkyl group, a C1 to C22 branched alkyl group, a C4 to C6 cycloalkyl group, or a C4 to C6 heterocycloalkyl group.

5. The method of preparing the ionic liquid of any one of claims 1 to 4, **characterized in that** the halohydrocarbon has a structure as shown in formula (2-1), and the sulfonyl halide has a structure as shown in formula (2-2),
R₁'-X₁ formula (2-1),
R₂'-SO₂X₂ formula (2-2),
wherein R₁' and R₂' are independently a C1 to C22 linear alkyl group, a C1 to C22 branched alkyl group, a C4 to C6 cycloalkyl group, a C4 to C6 heterocycloalkyl group, or a C6 to C10 aryl group, and X₁ and X₂ are independently fluorine, chlorine, bromine, or iodine.

6. The method of preparing the ionic liquid of any one of claims 1 to 5, **characterized in that** a reaction pressure of reacting the second compound with the lithium salt in the microwave device is greater than 1 atm and less than or equal to 2 atm.

7. The method of preparing the ionic liquid of any one of claims 1 to 6, **characterized by** further comprising:
after reacting the second compound with the lithium salt in the microwave device, performing a heating operation, wherein a heating temperature of the heating operation is 60 °C to 180 °C.

8. The method of preparing the ionic liquid of claim 7, **characterized in that** a heating time of the heating operation is less than or equal to 6 hours.

9. The method of preparing the ionic liquid of any one of claims 1 to 8, **characterized in that** a reaction temperature of reacting the halogen-containing compound with the first compound is 25 °C to 100 °C.

10. A method of preparing an ionic liquid, **characterized by** comprising:
reacting a halogen-containing compound with a first compound in a first microwave device to form a second compound, wherein the halogen-containing compound comprises a halohydrocarbon, a sulfonyl halide, or a combination thereof, the first compound comprises an amine compound having a tertiary amine group, a phosphine compound, or a combination thereof, the second compound comprises a first quaternary ammonium salt, a first quaternary phosphonium salt, or a combination thereof, and the amine compound having the tertiary amine group comprises a structure as shown in formula (1-1), an imidazole compound, a pyrrolidine compound, a pyridine compound, a pyrrole compound, a pyrazole compound, a structure as shown in formula (1-2), an imidazoline compound, a pyrazoline compound, or combinations thereof, 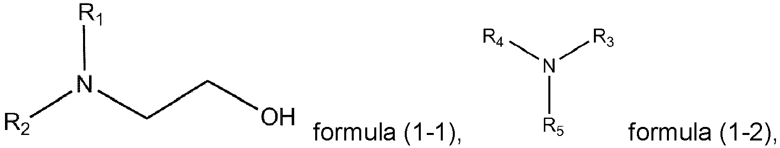 wherein R₁ to R₅ are independently a C1 to C22 linear alkyl group, a C1 to C22 branched alkyl group, a C4 to C6 cycloalkyl group, or a C4 to C6 heterocycloalkyl group; and
reacting the second compound with a lithium salt in a second microwave device to form a third compound, wherein the third compound comprises a second quaternary ammonium salt, a second quaternary phosphonium salt, or a combination thereof, and anions of the second compound and the third compound are different.

11. The method of preparing the ionic liquid of claim 10, **characterized in that** a microwave power of reacting the halogen-containing compound with the first compound in the first microwave device and a microwave power of reacting the second compound with the lithium salt reacted in the second microwave device are respectively 700 watts to 1400 watts.

12. The method of preparing the ionic liquid of any one of claims 10 to 11, **characterized in that** a reaction pressure of reacting the halogen-containing compound with the first compound in the first microwave device and a reaction pressure of reacting the second compound with the lithium salt in the second microwave device are respectively greater than 1 atm and less than or equal to 2 atm.

13. The method of preparing the ionic liquid of any one of claims 10 to 12, **characterized in that** a reaction temperature of reacting the halogen-containing compound with the first compound in the first microwave device and a reaction temperature of reacting the second compound with the lithium salt in the second microwave device are respectively 60 °C to 250 °C.

14. The method of preparing the ionic liquid of any one of claims 10 to 13, **characterized in that** the anion of the second compound is a halide ion or a structure as shown in formula (4-1), the anion of the third compound is bis(trifluoromethanesulfonyl)imide anion, bis(fluorosulfonyl)imide anion, tetrafluoroborate anion, hexafluorophosphate anion, trifluoromethanesulfonate anion, acetate ion, a structure as shown in formula (4-2), a structure as shown in formula (4-3), a structure as shown in formula (4-4), or combinations thereof, 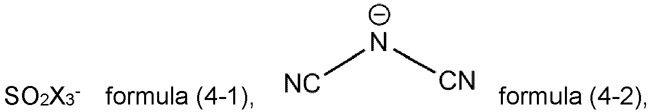 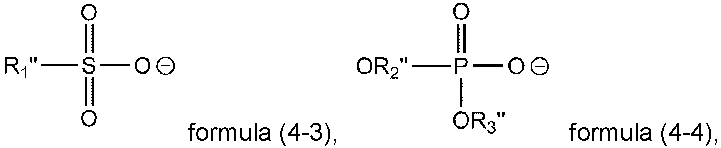 wherein X₃ is fluorine, chlorine, bromine, or iodine, and R₁", R₂", and R₃" are independently a C1 to C22 linear alkyl group, a C1 to C22 branched alkyl group, a C4 to C6 cycloalkyl group, or a C4 to C6 heterocycloalkyl group.

15. The method of preparing the ionic liquid of any one of claims 10 to 14, **characterized in that** a molar ratio of the halogen-containing compound to the first compound is 1:1 to 2:1, and a molar ratio of the second compound to the lithium salt is 1:1 to 1:2.
